# EUROPEAN PATENT SPECIFICATION

(11) **EP 4 406 605 B1**
(45) Date of publication and mention of the grant of the patent: **04.02.2026**
(21) Application number: 24166770.8
(22) Date of filing: 07.04.2021
(51) Int. Cl.: A61K 31/135, A61K 31/341, A61K 31/4164, A61K 31/426, A61K 31/495, A61K 45/06, A61P 11/00, A61P 31/14, A61P 31/16

(54) **METHODS AND PHARMACEUTICAL COMPOSITIONS FOR TREATING AND PREVENTING ACUTE RESPIRATORY DISTRESS SYNDROME ASSOCIATED WITH VIRAL INFECTIONS**
VERFAHREN UND PHARMAZEUTISCHE ZUSAMMENSETZUNGEN ZUR BEHANDLUNG UND PRÄVENTION VON AKUTEM ATEMNOTSYNDROM IM ZUSAMMENHANG MIT VIRUSINFEKTIONEN
PROCÉDÉS ET COMPOSITIONS PHARMACEUTIQUES POUR TRAITER ET PRÉVENIR LE SYNDROME DE DÉTRESSE RESPIRATOIRE AIGUË ASSOCIÉ À DES INFECTIONS VIRALES

(30) Priority: 07.04.2020 US 202063006599 P; 05.05.2020 US 202063020479 P; 22.06.2020 US 202063042434 P
(43) Date of publication of application: 31.07.2024
(62) Divisional of application: 21723469.9
(73) Proprietor: Hista RX LLC, Jackson, MS 39232 (US)
(72) Inventor: HOGAN II, Reed B., Jackson, 39202 (US)
(74) Representative: Barker Brettell LLP

(56) References cited:
- SIELAFF T D ET AL: "SUCCESSFUL TREATMENT OF ADULT RESPIRATORY DISTRESS SYNDROME BY HISTAMINE AND PROSTAGLANDIN BLOCKADE IN A PORCINE PSEUDOMONAS MODEL", SURGERY, MOSBY, INC, US, vol. 102, 1 January 1987 (1987-01-01), pages 350 - 357, XP000882601, ISSN: 0039-6060
- PATEL V ET AL: "ALL ROADS "LEAD" TO ANAPHYLAXIS: HYPERSENSITIVITY TO ELECTROCARDIOGRAM LEADS", ANNALS OF ALLERGY, ASTHMA & IMMUNOLOGY, vol. 121, no. 5, 2018, XP085539538, ISSN: 1081-1206, DOI: 10.1016/J.ANAI.2018.09.258
- DESHEVA YULIA ET AL: "Mucosal vaccine based on attenuated influenza virus and the group B Streptococcus recombinant peptides protected mice from influenza and S. pneumoniae infections", vol. 14, no. 6, 25 June 2019 (2019-06-25), pages e0218544, XP055813701, Retrieved from the Internet <URL:https://storage.googleapis.com/plos-corpus-prod/10.1371/journal.pone.0218544/1/pone.0218544.pdf?X-Goog-Algorithm=GOOG4-RSA-SHA256&X-Goog-Credential=wombat-sa@plos-prod.iam.gserviceaccount.com/20210614/auto/storage/goog4_request&X-Goog-Date=20210614T163505Z&X-Goog-Expires=86400&X-Goog-SignedHeaders=h> DOI: 10.1371/journal.pone.0218544
- HOGAN II REED B. ET AL: "Dual-histamine receptor blockade with cetirizine - famotidine reduces pulmonary symptoms in COVID-19 patients", vol. 63, 1 August 2020 (2020-08-01), GB, pages 101942, XP055780451, ISSN: 1094-5539, Retrieved from the Internet <URL:https://www.ncbi.nlm.nih.gov/pmc/articles/PMC7455799/pdf/main.pdf> DOI: 10.1016/j.pupt.2020.101942

## Description

### BACKGROUND

H1 and H2 receptor antagonists are two classes of antihistamines. H1 receptor antagonists (H1R) are used in the symptomatic treatment of multiple conditions, including allergic rhinoconjunctivitis, relief of pruritus in patients with urticaria, and in patients with chronic asthma. Newer H1 receptor antagonists, such as cetirizine, are referred to as second-generation H1 receptor antagonists, and are more selective for peripheral H1 receptors than first-generation H1 receptor antagonist, which antagonize both the central and peripheral nervous system H1 receptors as well as cholinergic (muscarinic) receptors. The selectivity significantly reduces the occurrence of adverse drug reactions, such as sedation, while still providing effective relief of allergic conditions.

H2 receptor antagonists (H2R) are used primarily to treat symptoms of acid reflux, or gastroesophageal reflux disease. H2 receptor antagonists reduce the production of stomach acid. Often diarrhea is listed as a major side effect of H2 receptor antagonists.

Diphenhydramine, a first-generation H1 receptor antagonist, together with either cimetidine or ranitidine, H2 receptor antagonists, have been studied for the treatment of acute allergic reactions. In a first study (Runge *et al.*,1992), patients were treated by a single intravenous administration of a solution 300 mg cimetidine and placebo, 50 mg diphenhydramine and placebo, or diphenhydramine plus cimetidine; the treatment was found effective for acute urticaria. In a second study (Lin *et al.*, . 2000), patients were treated by a single parenteral administration of a solution of either 50 mg diphenhydramine and saline or 50 mg diphenhydramine and 50 mg ranitidine. The treatment was found effective for acute allergic syndromes.

A combination histamine receptor blockade, a combination of 2 types of antihistamines, cetirizine (an H1R antagonist) and famotidine (an H2R antagonist) has been through FDA phase 1 trials for Irritable Bowel Syndrome (IBS) diarrhea predominate (IBS-d), a disease with histamine-driven pathogenesis. Previous studies from the Oklahoma Center for Neuroscience at the University of Oklahoma Health Sciences Center have documented the synergistic effect of this combination in specific ratios for nerve-mediated ion transport across the rat colonic mucosa and inhibition of visceral nociception due to stress. Double blinded placebo-controlled trials have established the synergistic effect of combination H1R and H2R antagonists for the treatment of IBS-d and idiopathic diarrhea. Another trial established the synergistic effect of combination H1R and H2R antagonists for the treatment of chemotherapy induced diarrhea. There is also anecdotal evidence of benefit in patients with HIV-induced diarrhea and *C. diff.* diarrhea. Currently this particular drug combination is awaiting initiation of FDA phase 2 trials.

Treatments and preventatives are needed for COVID-19, the disease that is caused by SARS-CoV-2. This virus has caused a pandemic that has led to more than three million confirmed cases of infections worldwide and more than 200,000 deaths worldwide, as of the end of April, 2020. Unfortunately, this virus continues to spread globally and is projected to cause perhaps hundreds of thousands of additional deaths, if no effective interventions are found and used.

The number of infected individuals worldwide is highly likely to be considerably higher than the number of RT-PCR Positive confirmed cases to date, perhaps by an order of magnitude. It will take antibody testing of random representative populations to estimate the incidence of retroviral infection in geographic regions. But, these antibody studies have lagged behind RT-PCR testing that has primarily emphasized only moderate-to-severe symptomatic patients.

New drug candidates (and vaccine candidates) are being developed for COVID-19, but their efficacies and safeties are still uncertain, and they are expected to take at least a year or more to become commercially available. An effective drug that is already known to be safe for human use would provide an ideal treatment. Thus, a re-purposed drug or drug combination can be made available quickly, provided the active pharmaceutical ingredients (APIs) have been approved in at least one formulation.

Pulmonary pathology in early-phase COVID-19 pneumonia has shown exudative and proliferative phases of acute lung injury (edema, inflammatory infiltrates, pneumocyte hyperplasia) prior to the development of any respiratory symptoms. In the later stage of disease, patients can develop acute respiratory distress syndrome (ARDS) and multi-organ failure. ARDS has taken center stage as the primary cause of death in the global COVID-19 crisis. The "cytokine storm" is emerging as a key mechanism leading to patient deterioration and death. Cytokine storm and ARDS has been associated with death from other viral infections, including SARS (SARS-COV) and influenza viruses, of which the Spanish flu virus caused the 1918 pandemic that claimed millions of lives.

Cytokine storm is associated with marked morbidity and mortality in patients presenting during the COVID-19 pandemic. The overwhelming progression to pulmonary failure is the hallmark vicious cycle overwhelming worldwide healthcare resources. Even a modest improvement in decreasing ventilator dependence, for example 5-10%, could dramatically alter outcomes in many healthcare settings. Even reducing the rate of hospital admissions, for example 5-10%, could dramatically alter outcomes (i.e., preventing hospitalizations would prevent subsequent ventilator dependence).

### SUMMARY

In a first aspect, the present invention relates to a product comprising an H1 receptor antagonist and an H2 receptor antagonist for use in a method of: (a) treating or preventing acute respiratory distress syndrome or pulmonary distress in a patient; or (b) preventing pulmonary-related hospitalization, pulmonary intubation, or pulmonary failure in a patient; or (c) treating or preventing a pulmonary cytokine storm in a patient; wherein the product is administered to a patient infected with a corona virus or an influenza virus; wherein the H1 receptor antagonist comprises cetirizine, levocetirizine, diphenhydramine, or mixtures thereof and wherein the H1 receptor antagonist is administered in an amount of 1.0 to 14 mg per dose, such as a dosage of 6.0 to 14 mg; and wherein the H2 receptor antagonist comprises famotidine and wherein the H2 receptor antagonist is administered in an amount of 5.0 to 28 mg per dose, such as a dosage of 10 to 28 mg.

In a second aspect, the present invention relates to a product comprising an H1 receptor antagonist for use in a method of: (a) treating or preventing acute respiratory distress syndrome or pulmonary distress in a patient; or (b) preventing pulmonary-related hospitalization, pulmonary intubation, or pulmonary failure in a patient; or (c) treating or preventing a pulmonary cytokine storm in a patient, wherein the H1 receptor antagonist is administered to a patient infected with a corona virus or an influenza virus, and wherein the patient is further administered an H2 receptor antagonist, wherein the H1 receptor antagonist comprises cetirizine, levocetirizine, diphenhydramine, or mixtures thereof and wherein the H1 receptor antagonist is administered in an amount of 1.0 to 14 mg per dose, such as a dosage of 6.0 to 14 mg; and wherein the H2 receptor antagonist comprises famotidine and wherein the H2 receptor antagonist is administered in an amount of 5.0 to 28 mg per dose, such as a dosage of 10 to 28 mg.

In a third aspect, the present invention relates to a product comprising an H2 receptor antagonist for use in a method of: (a) treating or preventing acute respiratory distress syndrome or pulmonary distress in a patient; or (b) preventing pulmonary-related hospitalization, pulmonary intubation, or pulmonary failure in a patient; or (c) treating or preventing a pulmonary cytokine storm in a patient, wherein the H2 receptor antagonist is administered to a patient infected with a corona virus or an influenza virus, and wherein the patient is further administered an H1 receptor antagonist, wherein the H1 receptor antagonist comprises cetirizine, levocetirizine, diphenhydramine, or mixtures thereof and wherein the H1 receptor antagonist is administered in an amount of 1.0 to 14 mg per dose, such as a dosage of 6.0 to 14 mg; and wherein the H2 receptor antagonist comprises famotidine and wherein the H2 receptor antagonist is administered in an amount of 5.0 to 28 mg per dose, such as a dosage of 10 to 28 mg.

Further embodiments of the invention are defined in the appended claims.

### DEFINITIONS

The term "unit dosage form," means a single pre-measured dose, and includes tablets, pills, capsules, packets, suspensions, transdermal patches, rectal suppositories, and sterile pre-measured liquid formulations ready for injection.

H1R means histamine type-1 receptor.

H2R means histamine type-2 receptor.

ARDS means acute respiratory distress syndrome.

OTC means over-the-counter.

IV means intravenous administration.

PO means oral administration.

"q" relates to frequency of administration.

RT-PCR means Reverse Transcriptase - Polymerase Chain Reaction.

### DETAILED DESCRIPTION

The present invention makes use of administering an H1 receptor antagonist and an H2 receptor antagonist in combination to a patient, to provide prevention or a significant reduction in pulmonary failure associate with viral infections, to treat acute respiratory distress syndrome (ARDS), such as infection with SARS-COV-2 (also referred to as COVID-19), SARS-COV, other corona viruses, and influenza viruses. The H1receptor antagonist according to the invention comprises cetirizine, levocetirizine, diphenhydramine or mixtures thereof. Preferred H2 receptor antagonists include famotidine and ranitidine. The H2 receptor antagonist according to the invention comprises famotidine. Administration orally (p.o.) is preferred, if the patient is able to take oral medication, otherwise IV or gastric via nasogastric tube administration is preferred.

Dual-histamine blockade has shown significant efficacy in multiple clinical applications and peer-reviewed studies. COVID-19 is known to cause pulmonary disease with a wide range of severity. Patients have been found to have significant pulmonary disease on radiographic imaging and pathology specimens even before exhibiting any pulmonary symptoms. The cytokine storm, mediated in part by histamine, is thought to be a key mechanism of action resulting in patient deterioration. It is reasonable to consider the use of dual-histamine blockade to stop the histamine-cytokine network and blunt the cytokine storm.

The safety of these antihistamine agents is also advantageous. The historic safety of selected H1R and H2R antagonists (e.g., cetirizine, diphenhydramine, famotidine) enabled formulations with these active pharmaceutical ingredients (APIs) to be regulated as over-the-counter (OTC) medications. For instance, cetirizine is a US OTC at 10 mg, diphenhydramine is a US OTC at 25 mg, and famotidine is a US OTC at 10 or 20 mg. The OTC safety designations, coupled to the efficacy and safety profile of dual-histamine blockade in a variety of contexts in animal models and humans to date, makes this an appealing consideration for all patients who test positive for COVID-19 by Reverse Transcriptase - Polymerase Chain Reaction (RT-PCR).

Furthermore, the safety profile of the preferred histamine receptor antagonists in this combination drug approach provide distinct advantages over the rapidly-evolving clinical standard(s)-of-care treatments (e.g., medications and/or biologics and/or hospitalization and/or ventilation) for COVID-19 during the ongoing global pandemic. If dual-histamine blockade is able to blunt the cytokine storm within the lungs, it is hypothesized that the severity of disease would lessen before, during, and after hospitalization, and hospitalization admissions and duration of in-patient hospitalization would lessen, and ventilator use would decrease. Reducing severity of disease and reducing the need for ventilators would have a tremendous impact on our global and local healthcare infrastructure and would save many lives during this pandemic. A safe and effective approach is facilitated by the dual-histamine blockade for pulmonary pathogenesis, relative to current standard(s)-of-care.

The histamine-cytokine network has been studied extensively and is well defined in the literature. Histamine modulates the release of cytokines. Histamine is known to exert pro-inflammatory effects (e.g., increased local blood flow and capillary permeability) and to influence local immune responses in the lungs (e.g., cytokine release). These effects lead to pulmonary tissue damage.

Animal model studies have shown successful treatment of ARDS with therapies that include dual histamine blockade. Studies have looked at combinations of histamine and prostaglandin blockade in ARDS models. Interestingly, when a combination of H1R + H2R + cyclooxygenase inhibitor + steroid + antihypertensives were studied in ARDS models, it was the dual-histamine blockade that proved to be the most beneficial. That study concluded that dual-histamine blockade + ibuprofen was essential in the treatment of hypoxemia, pulmonary hypertension, and pulmonary microvascular injury in this fulminant ARDS model (Sielaff, T.D., et al., 1987).

The present invention also includes unit dosage forms, multi-dosage forms, and kits, including an H1 receptor antagonist and an H2 receptor antagonist. Preferably, the H1 receptor antagonist includes cetirizine and the H2 receptor antagonist includes famotidine. The combination may be an oral dosage form for ingestion, or a gastric dosage form for delivery via a nasogastric tube, a mucosal dosage form, or an injectable dosage form (e.g., IV).

H1 receptor antagonists block H1 histamine receptors; first-generation H1 receptor antagonists block histamine receptors in the central and peripheral nervous systems, as well as cholinergic (muscarinic) receptors, while second-generation H1 receptor antagonists are selective for H1 histamine receptors in the peripheral nervous system. First-generation H1 receptor antagonists include brompheniramine, chlorpheniramine, dexbrompheniramine, dexchlorpheniramine, pheniramine, triprolidine, carbinoxamine, clemastine, diphenhydramine, pyrilamine, promethazine, hydroxyzine, azatadine, cyproheptadine, and phenindamine. Second-generation H1 receptor antagonists include ketotifen, rupatadine, mizolastine, acrivastine, ebastine, bilastine, bepotastine, terfenadine, quifenadine, azelastined, cetirizine, levocetirizine, desloratadine, fexofenadine and loratadine. Preferably, the H1 receptor antagonist is a second-generation H1 receptor antagonist, more preferably the H1 receptor antagonist is cetirizine or levocetirizine, with cetirizine being particularly preferred. Mixtures and combination of H1 receptor antagonists may also be used.

Prescription generic and over-the-counter (OTC) products containing at least one H1R antagonist are currently FDA approved and commercially-available. Prescription generic and/or OTC products containing at least one H1R antagonist may be preferred for specific medical conditions of the present invention. For instance, generic and/or OTC products may be preferred for outpatient treatments.

The H1 receptor antagonists may be used in an amount of from 0.1 to 10 times the amount typically used for the treatment of allergies, for example in an amount of 0.1 to 600 mg per dose, 0.5 to 500 mg per dose, 1.0 to 50 or 60 mg per dose, including 1.25, 1.5, 1.75, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, 20, 25, 30, 35, 40 and 45 mg per dose. Preferably, the H1 receptor antagonist is administered 1, 2, 3 or 4 times per day. The H1 receptor antagonist may also be administered PRO RE NATA (as needed). The H1 receptor antagonist may be administered as an injectable formulation, for example intravenously, intraparenterally or intramuscularly; transdermally, via a transdermal patch; or, preferably, orally, as a powder, table or capsule, an oral solution or suspension, or sublingual or buccal tablets; or preferably a solution or suspension via nasogastric tube. Alternative forms of administration include rectal suppositories, inhaled, epidural, subcutaneous, nasal spray, transmucosal, and intradermal formulations.

H2 receptor antagonists block H2 histamine receptors. H2 receptor antagonists include cimetidine, ranitidine, famotidine, and nizatidine. The claimed invention requires that the H2 receptor antagonist comprises famotidine. Mixtures and combinations of H2 receptor antagonists may also be used.

Prescription generic and over-the-counter (OTC) products containing at least one H2R antagonist are currently FDA approved and commercially-available. Prescription generic and/or OTC products containing at least one H2R antagonist may be preferred for specific medical conditions of the present invention. For instance, generic and/or OTC products may be preferred for outpatient treatments.

The H2 receptor antagonists may be used in an amount of from 0.1 to 10 time the amount typically used for treatment dyspepsia, for example 1.0 to 8000 mg per dose, 2.0 to 1000 mg per dose, 5.0 to 800 mg per dose, including 6.0, 7.0, 8.0, 9.0, 10, 15, 20, 21, 22, 22.5, 23, 24, 25, 26, 27, 28, 29, 30, 35, 40, 45, 50, 55, 60, 65, 70, 75, 80, 85, 90, 95, 100, 120, 140, 150, 175, 200, 250, 300, 350, 400, 450, 500, 600, and 700 mg per dose. Preferably, the H2 receptor antagonist is administered 1, 2, 3 or 4 times per day. The H2 receptor antagonist may also be administered PRO RE NATA (as needed). The H2 receptor antagonist may be administered as an injectable formulation, for example intravenously, intraparenterally or intramuscularly; transdermally, via a transdermal patch; or, preferably, orally, as a powder, table or capsule, an oral solution or suspension, or sublingual or buccal tablets; or preferably a solution or suspension via nasogastric tube. Alternative forms of administration include rectal suppositories, inhaled, epidural, subcutaneous, nasal spray, transmucosal, and intradermal formulations.

Patients may respond to treatment within 4 to 72 hours. However, treatment should be carried out for an amount of time to resolve the pulmonary distress, for example 3 to 30 days, or 5 to 14 days. Treatment may be initiated at the first signs of infection, prior to symptoms upon the indication of the presence of a virus by RT-PCR diagnostic testing, delayed until the first signs of pulmonary distress, or at any point prior to death.

Preferably, the H1 and H2 receptor antagonists are administered together or simultaneously, and as a unit dosage form containing both receptor antagonists. Examples of unit dosage forms include oral compositions, such as tablets (for example, sublingual or buccal tablets), orally-disintegrating tablets (ODTs), capsules (for example, hard gelatin and soft gelatin capsules), transmucosal and sublingual patches and films, pre-measured powder packets and sachets, flavored and/or sweetened aqueous solutions or suspensions. Preferably, the unit dosage form is present as a once-per-day dosage, although other dosage schedules are envisioned (e.g., twice-per-day or PRO RE NATA).

Other unit dosage forms may also be provided, containing both H1 and H2 receptor antagonist. For example, injectable formulation containing a sterile solution or suspension, including formulation for administration intravenously, intraparenterally or intramuscularly, may be provided. Injectable formulation, such as a sterile premeasured single dose read-to-inject form is also a unit dosage form. A unit dosage of a solution or suspension may be delivered by nasogastric tube into the stomach. A unit dosage form for administration transdermally, via a transdermal patch, may be provided. Other unit dosage forms include rectal suppositories, inhaled, epidural, subcutaneous, nasal spray, and intradermal formulations.

Pharmaceutically-acceptable excipients and adjuvants (e.g., buffers, surfactants, lipophilic agents, sugars, alcohols, solubilizers, bonding agents, dispersion agents, and/or pharmaceutically-acceptable salts thereof) may also be included in any of the pharmaceutical compositions or unit dosage forms, both oral and non-oral.

Multi-dosage forms, such as kits, containing 2 to 30, 3 to 25, or 5 to 14 unit dosage forms, for example 6, 7, 8, 9, 10, 11, 12, 13, 15, 20, 40, 50 or 60 unit dosage forms, may be provided. Preferably, the multi-dosage forms contain sufficient unit dosage forms for administration over a period of 2 to 30, 3 to 25, or 7 to 14 days, for example 4, 5, 6, 7, 8, 9, 10, 11, 12, 13, 20 or 30 days. Kits may also be provided, which include oral rehydration solutions, or powders which may be hydrated to form oral rehydration solutions, or kits containing sodium and glucose or a glucose-containing saccharide, as well as other pharmaceutically-acceptable excipients, flavorings and/or sweeteners, buffers, together with unit dosage forms.

In the event that FDA-approved finished formulations are not available, then prescription medications may be prepared by compounding pharmacy subject to 503A or 503B regulations.

Histamine H1 receptor antagonists include but are not limited to acrivastine, alimemazine, antazoline, astemizole, azatadine, azelastine, bamipine, bromazine, brompheniramine, buclizine, carbinoxamine, cetirizine, chlorcyclizine, chloropyramine, chlorphenamine, chlorpheniramine, chlorphenoxamine, cinnarizine, clemastine, cyclizine, cyproheptadine, deptropine, desloratadine, dexbrompheniramine, dexchlorpheniramine, dimenhydrinate, dimetindene, diphenhydramine, diphenylpyraline, dithiaden, doxepin, doxylamine, ebastine, emedastine, epinastine, fexofenadine, histapyrrodine, hydroxyethylpromethazine, hydroxyzine, isothipendyl, ketotifen, levocabastine, levocetirizine, loratadine, mebhydrolin, meclizine, meclozine, medizine, mepyramine, mequitazine, methapyrilene, methdilazine, mizolastine, mizolastine, olopatadine, oxatomide, oxomemazine, phenindamine, pheniramine, pimethixene, promethazine, pyrilamine, pyrrobutamine, rupatadine, talastine, terfenadine, thenalidine, tripelennamine, and triprolidine.

The claimed invention requires that the H1 receptor antagonist comprises cetirizine, levocetirizine, diphenhydramine, or mixtures thereof.

Histamine H2 receptor antagonists include but are not limited to cimetidine, famotidine, nizatidine, ranitidine, roxatidine, ebrotidine, lafutidine, niperotidine, potentidine, and zolantidine. The claimed invention requires that the H2 receptor antagonist comprises famotidine.

### EXAMPLES

### Clinical Trials of Dual-Histamine Receptor Blockade in Viral-infected Patients Purpose

To demonstrate dual-histamine receptor blockade to blunt the cytokine storm in the acute COVID-19 infectious process in subjects ill enough (severe) to be admitted to a hospital for observation. Depending on the density of COVID-19 infections, at least 1 to 200 patients will be enrolled. The principle clinical questions are: Can the deterioration in pulmonary function and mortality be impacted by blunting the cytokine storm with dual-histamine receptor blockade? Can blunting the cytokine storm shorten hospital stay, reduce the number of patients intubated or the duration of ventilator dependence, and reduce mortality rates? Can treatments and preventatives be effective in two contexts, inpatient and outpatient environments? Can the treatments and preventatives be demonstrated to be superior to standard-of-care alone or standard-of-care in combination with experimental therapies (e.g., pharmaceuticals, biologics, or non-medical treatment modalities)?

### Objective

To decrease the progression of acutely ill (moderate-to-severe) hospitalized COVID-19 patients to ventilation and terminal event by blocking the cytokine storm (cytokine cascade), mediated in part by histamine. And, to reduce deaths, and reduce advancement to ventilation. Furthermore, to provide a means of treatment or prophylaxis in infected patients with no or minimal pulmonary symptoms, especially in an outpatient environment.

### Study compounds

The H1R antagonist is cetirizine and/or diphenhydramine.

The H2R antagonist is famotidine.

### Study population:

**Treatment Groups A & B (Inpatient):** Patients admitted to one or more medical centers (hospitals) with acute pulmonary illness clinically suspected of being COVID-19. Due to disease symptom severity these hospitalized patients are likely to be treated in Intensive Care Units (ICUs) and/or COVID wards. The likely routes of administration are oral if feasible, and if not feasible then preferably gastric via nasogastric tube and/or injection (e.g., IV). Group A is dual-histamine blockade and Group B is the control without dual-histamine blockade.

### Sample Size

Sample size will be between approximately 20-100 patients in each arm depending on recruitment and accumulation rates and data analysis.

### Study Duration

The length of time to enroll a maximum of 200 patients is estimated at 6 months in a single medical center or metropolitan region. The course of inpatient treatment per patient is estimated to last from approximately 1 to 30 days.

### Methodology

Given the current challenge of market availability of oral H2 antagonists, the inventors suggest using famotidine IV. Cetirizine is preferred as the H1 antagonist. However, the clinician may select diphenhydramine as the H1 antagonist, as it is available in IV form. However, the latter is more sedating than cetirizine.

In the event that FDA-approved finished formulations (generic or branded products) are not available, then prescription medications may be prepared by compounding pharmacy subject to 503A or 503B regulations.

**Treatment Group A:** On admission to hospital, the patient is preferably located within a COVID ward, and treatment is initiated in ER with standard-of-care per admitting provider. Confirm that the patient is Positive for COVID-19 by RT-PCR diagnostic test.

### Initiate Study Drug

Administer the first dose in the ER or upon arrival to the floor (e.g., COVID ward).

Famotidine 20 mg IV and Cetirizine 10 mg IV if available (or alternatively PO). However, if the patient is unable to take PO Cetirizine, then substitute diphenhydramine 25 or 50 mg IV.

Administer the subsequent doses of Famotidine 20 mg q 12 hrs and Cetirizine 10 mg q 12 hrs PO.

If the patient is intubated or NPO (nothing by mouth), then switch to IV H1R antagonist + IV H2R antagonist combination treatment, using Famotidine 20 mg IV q 12h and Diphenhydramine 25 or 50 mg IV q 12h.

The likely routes of antihistamine administration are oral when feasible, and if not feasible then gastric via nasogastric tube and/or injection (e.g., IV).

**Treatment Group B:** The control arm will receive standard-of-care treatment per admitting team, no study medications (i.e., no H1 antagonist and no H2 antagonist), 1:1 age stratify relative to Group A patient population.

### Exclusions for Treatment Group A & B

The patient is Negative for COVID-19 by RT-PCR diagnostic test.

Admission to ICU bed or ventilated within 24 hours of admission.

Sensitivity or allergy to H1 receptor antagonists or H2 receptor antagonists.

The patient has a Do Not Resuscitate (DNR) instruction.

### Primary Study Endpoints:

Reduce Ventilation requirements for group A vs group B.

Reduce Mortality rates in group A vs group B.

Shorten Hospital day for group A vs group B.

Each of the preceding study endpoints compared to published standard-of-care patient outcomes without the dual-histamine blockade medications, either as the control arms of the published studies or as the medication treatment arms (e.g., a single histamine receptor antagonist, and/or other pharmaceuticals, and/or biologics) of the published studies. This type of comparison may be especially informative if the number of patients is limited in the control group B, due to compassionate care or Institutional Review Board considerations during the rapidly-evolving standard(s)-of-care in the COVID-19 pandemic. Thus, this endpoint (group A vs published standard-of-care groups) may serve as an alternative to a direct group A vs group B comparison.

### Secondary Study Endpoints:

Time from admission till intubation for group A vs group B.

Time from admission till death for group A vs group B.

Time from intubation till death for group A vs group B.

Time from intubation till extubation for group A vs group B. Time from admission till discharge for group A vs group B.

Time to resolution of pulmonary symptoms for group A vs group B.

Lowest O₂ concentration (nadir) during admission or prior to intubation for group A vs group B.

Time from admission till blood O₂ concentration is normal (or > 90 percent) for group A vs group B.

Stratify by age and comorbidities, explore possible positive treatment groups for group A vs group B.

Each of the preceding study endpoints compared to published standard-of-care patient outcomes without the dual-histamine blockade medications (or single histamine receptor antagonist), either as the control arms of the published studies or as the medication treatment arms (e.g., a histamine receptor antagonist alone, and/or other pharmaceuticals, and/or biologics) of the published studies. This type of comparison may be especially informative if the number of patients is limited in the control group B, due to compassionate care or Institutional Review Board considerations during the rapidly-evolving standard(s)-of-care in the COVID-19 pandemic. Thus, this endpoint (group A vs published standard-of-care groups) may serve as an alternative to a direct group A vs group B comparison.

### Results of Group A

Using a design similar to or approximating the Group A description (above), in recent months at least 109 COVID-19-positive patients with pulmonary symptoms have been treated in an inpatient setting with an H1R antagonist + H2R antagonist combination (cetirizine + famotidine), in addition to standard-of-care treatments. A case series of 109 COVID-19-positive inpatients with severe to critical pulmonary symptoms were treated with cetirizine 10 mg b.i.d. plus famotidine 20 md b.i.d. for at least 48 hours. Of all patients, including those with Do Not Resuscitate directives, receiving the dual-histamine blockade for at least 48 hours, the combination drug treatment resulted in a 15.6% rate of intubation, a 7.3% rate of intubation after 48 hours of treatment, a 15.6% rate of inpatient mortality, and 10.9 days duration of hospitalization (see Table below). The drug combination exhibited reductions in symptom progression when compared to published reports of COVID-19 patients.

**Table II: Clinical outcomes in 109 COVID-19 inpatients treated with famotidine and cetirizine for at least 48 hours**

| Key Metric: | | Including DNR | Excluding DNR |
|---|---|---|---|
| Total Patients Admitted | | 109 | 96 |
| Total Patients Discharged | | 92 | 88 |
| | Discharge Rate | 84.4% | 91.7% |
| Total Patients Intubated | | 17 | 15 |
| | Intubation Rate | 15.6% | 15.6% |
| Total Patients Intubated after 48 hrs of Treatment | | 8 | 6 |
| | Intubation Rate after 48 hrs of Treatment | 7.3% | 6.3% |
| Total Deaths | | 17 | 8 |
| | Death Rate % | 15.6% | 8.3% |
| Average Days to Discharge | | 10.9 | 10.9 |

Given (a) the recent emergence of COVID-19 in China in late 2019 and in the USA in early 2020, (b) the rapid need for safe and effective treatments deployable immediately to clinics, and (c) the rapid evolution in the standard(s)-of-care that are influenced by media reporting, recent innovations in clinical therapies (including the present invention) may not permit sufficient time for the statistically robust clinical trials that are customary for an FDA regulatory approval process.

In this time-is-of-the-essence global pandemic context, the results of the dual-histamine receptor blockade method of treatment and prophylaxis compare favorably to current standard-of-care patients not receiving dual-histamine receptor blockade therapy, which may serve as highly relevant external control groups (and in lieu of Group B, if necessary). Outside of this current invention, a range of 26% to 32% of hospitalized COVID-19 patients were subsequently admitted into Intensive Care Units (ICU). And, estimates of mortality in ICU patients range from 39% to 72% (www.cdc.gov/coronavirus/2019-ncov/hcp/clinical-guidance-management-patients.html).

The present invention addresses this time-sensitive unmet medical need, and provides effective and safe means to rapidly affect patient outcomes in both inpatient and outpatient environments, which are anticipated to save lives. The highly favorable circumstances of having commercially-available OTC and generic antihistamine drugs of both receptor types provides another distinct advantage to the present invention relative to other experimental drug and biologic research programs (e.g., novel molecular entities), some of which may take numerous years to develop. The present invention may be deployed immediately or quickly in clinics, without any need for delays.

### Treatment Group C (Outpatient)

Depending on availability of patients with no or mild symptoms that are confirmed Positive for COVID-19 by RT-PCR diagnostic test, and a beneficial treatment response in treatment group A relative to a control group B or alternative control group from publications, a subsequent outpatient study is anticipated. The patients will self-administer the dual-histamine combination (and/or coincident use of two formulations) as an oral prophylactic or treatment. The vast majority of these patients are not expected to be admitted to a hospital in view of symptom severity. Depending upon IRB guidance, this may be viewed as a compassionate-care use and/or a "new" standard-of-care treatment for outpatients. This may alternatively be performed as a physician-sponsored study (without IRB review), given that OTC antihistamine products are readily available. The control group(s) could be either a placebo or relative to published control arms without experimental treatments.

### Sample Size

Sample size will be between approximately 20-100 patients in each arm depending on recruitment and accumulation rates and data analysis.

### Study Duration

The length of time to enroll a maximum of 200 patients is estimated at 6 months in a single medical center or metropolitan region. The course of outpatient treatment per patient is estimated to last from approximately 1 to 30 days.

### Methodology

Oral cetirizine is preferred as the H1R antagonist. However the clinician may select oral diphenhydramine as an alternative H1R antagonist. However, the latter is more sedating than cetirizine. Oral famotidine is preferred as the H2R antagonist.

In the event that FDA-approved finished formulations (generic or branded products) are not available, then prescription medications may be prepared by compounding pharmacy subject to 503A or 503B regulations.

### Treatment of Group C

The patient is enrolled in the study on an outpatient basis. Confirm that the patient is Positive for COVID-19 by RT-PCR diagnostic test.

### Initiate Study Drug

One or more arms may include OTC products co-administered or in a dual drug combination. Cetirizine is available as a US OTC at 10 mg, diphenhydramine is available as a US OTC at 25 mg, and famotidine is available as a US OTC at 10 or 20 mg. The treatment or prevention would involve co-administration (coincident use of two formulations) or a dual drug combination of at least one H1R antagonist (e.g., cetirizine 10 mg and/or diphenhydramine 25 mg) plus at least one H2R antagonist (e.g., famotidine 10 or 20 mg).

Alternatively, FDA-approved prescription branded or generic drug formulations may be used. In addition, compounded products (e.g., dual drug combination) produced under 503A or 503B regulations may also be used

### Exclusions for Treatment Group C

The patient is Negative for COVID-19 by Reverse Transcriptase - Polymerase Chain Reaction (RT-PCR) diagnostic test.

Admission to a hospital for severe pulmonary symptoms (e.g., ARDS) within prior 14 days.

Sensitivity or allergy to H1 receptor antagonists or H2 receptor antagonists.

The patient has a Do Not Resuscitate (DNR) instruction.

### Primary Study Endpoints

The study endpoints for outpatient treatment would emphasize prevention of disease progression to severity. Examples of endpoints include:

Reduce admissions to hospital in Group C vs control Group.

Reduce pulmonary symptoms (severity, number of, and/or duration) vs. control Group.

Lowest blood O₂ concentration (nadir) vs. control Group.

In patients that progressed in symptom severity requiring hospitalization, all of the primary and secondary endpoints used for Groups A and B (above) could also be assessed as secondary endpoints for Group C.

In addition to a placebo control group (if permitted by IRB guidance), then alternative control group(s) may be obtained from published standard-of-care patient outcomes without the dual-histamine blockade medications, either as the control arms of the published studies or the medication treatment arms (e.g., a single histamine receptor antagonist) of the published studies. This type of comparison may be especially informative if the number of patients is limited in the placebo control group, due to compassionate care or Institutional Review Board considerations during the rapidly-evolving standard(s)-of-care in the COVID-19 pandemic.

### REFERENCES

Sielaff, T.D., et al. "Successful Treatment of Adult Respiratory Distress Syndrome by Histamine and Prostaglandin Blockade in a Porcine Pseudomonas Model" Surgery 102(2) 350-357 (Aug. 1987).
H2 blockers, MedlinePlus®, U.S. National Library of Medicine, NIH, updated: August 11, 2011.
Runge et al. "Histamine antagonists in the treatment of acute allergic reactions" Ann Emerg Med (Mar. 1992) 21:237-242.
Lin et al. "Improved outcomes in patients with acute allergic syndromes who are treated with combined H1 and H2 antagonists" Ann Emerg Med (Nov. 2000) 36:462-468.
He, Shuang; Li, Feng; Zhou, Dan; Du, Junrong; Huang, Yuan, Drug development and industrial pharmacy, (Oct. 2012) 38(10)1280 -1289.
Akin C, Valent P, Metcalfe DD "Mast cell activation syndrome: Proposed diagnostic criteria" J Allergy Clin Immunol. (2010) 126(6):1099-104.
Hamilton MJ, Hornick JL, Akin C, Castells MC, Greenberger NJ "Mast cell activation syndrome: a newly recognized disorder with systemic clinical manifestations" J Allergy Clin Immunol. (2011) 128(1):147-152.
Valent P "Mast cell activation syndromes: definition and classification" Allergy (2013) [Epub ahead of print 15 Feb. 2013].
Conti P, Ronconi G, Caraffa A, Gallenga CE, Ross R, Frydas I. Induction of pro-inflammatory cytokines (IL-1 and IL-6) and lung inflammation by Coronavirus-19 (COVI-19 or SARS-CoV-2): anti-inflammatory strategies. J. Biol. Regul. Homeost. Agents. 2020;34
Zhang W, Zhao Y, Zhang F, Want Q, LiT, Liu Z, Want J, Qin Y, Zhang X, Yan X, Zeng X, Zhang S. The use of anti-inflammatory drugs in the treatment of people with severe coronavirus disease 2019 (COVID-19): The Perspectives of clinical immunologists from China. Clin Immunol. 2020 Mar 25;214:108393.
Branco AC, Yoshikawa FY, Pietrobon AJ, Sato MN. Role of Histamine in Modulating the Immune Response and Inflammation. Mediators Inflamm. 2018 Aug 27;2018:9524075.
Zhao J, Zhao J, Perlman S. T cell responses are required for protection from clinical disease and for virus clearance in severe acute respiratory syndrome coronavirus-infected mice. J Virol. 2010;84(18):9318-9325. doi:10.1128/JVI.01049-10
Kmiecik T, Otocka-Kmiecik A, Górska-Ciebiada M, Ciebiada M. T lymphocytes as a target of histamine action. Arch Med Sci. 2012;8(1):154-161.
Jang Y, Jin M, Seo SH. Histamine contributes to severe pneumonia in pigs infected with 2009 pandemic H1N1 influenza virus. Arch Virol. 2018 Nov;163(11):3015-3022.

## Claims

1. A product comprising an H1 receptor antagonist and an H2 receptor antagonist for use in a method of: (a) treating or preventing acute respiratory distress syndrome or pulmonary distress in a patient; or (b) preventing pulmonary-related hospitalization, pulmonary intubation, or pulmonary failure in a patient; or (c) treating or preventing a pulmonary cytokine storm in a patient;
wherein the product is administered to a patient infected with a corona virus or an influenza virus;
wherein the H1 receptor antagonist comprises cetirizine, levocetirizine, diphenhydramine, or mixtures thereof and wherein the H1 receptor antagonist is administered in an amount of 1.0 to 14 mg per dose, such as a dosage of 6.0 to 14 mg; and
wherein the H2 receptor antagonist comprises famotidine and wherein the H2 receptor antagonist is administered in an amount of 5.0 to 28 mg per dose, such as a dosage of 10 to 28 mg.

2. A product comprising an H1 receptor antagonist for use in a method of: (a) treating or preventing acute respiratory distress syndrome or pulmonary distress in a patient; or (b) preventing pulmonary-related hospitalization, pulmonary intubation, or pulmonary failure in a patient; or (c) treating or preventing a pulmonary cytokine storm in a patient,
wherein the H1 receptor antagonist is administered to a patient infected with a corona virus or an influenza virus, and wherein the patient is further administered an H2 receptor antagonist,
wherein the H1 receptor antagonist comprises cetirizine, levocetirizine, diphenhydramine, or mixtures thereof and wherein the H1 receptor antagonist is administered in an amount of 1.0 to 14 mg per dose, such as a dosage of 6.0 to 14 mg; and
wherein the H2 receptor antagonist comprises famotidine and wherein the H2 receptor antagonist is administered in an amount of 5.0 to 28 mg per dose, such as a dosage of 10 to 28 mg.

3. A product comprising an H2 receptor antagonist for use in a method of: (a) treating or preventing acute respiratory distress syndrome or pulmonary distress in a patient; or (b) preventing pulmonary-related hospitalization, pulmonary intubation, or pulmonary failure in a patient; or (c) treating or preventing a pulmonary cytokine storm in a patient,
wherein the H2 receptor antagonist is administered to a patient infected with a corona virus or an influenza virus, and wherein the patient is further administered an H1 receptor antagonist,
wherein the H1 receptor antagonist comprises cetirizine, levocetirizine, diphenhydramine, or mixtures thereof and wherein the H1 receptor antagonist is administered in an amount of 1.0 to 14 mg per dose, such as a dosage of 6.0 to 14 mg; and
wherein the H2 receptor antagonist comprises famotidine and wherein the H2 receptor antagonist is administered in an amount of 5.0 to 28 mg per dose, such as a dosage of 10 to 28 mg.

4. The product for use according to any one of the preceding claims, wherein the use is treating acute respiratory distress syndrome or pulmonary distress in the patient.

5. The product for use according to any one of the preceding claims, wherein the use is treating a pulmonary cytokine storm in the patient.

6. The product for use according to any one of the preceding claims, wherein the patient is infected with SARS-COV or SARS-COV-2.

7. The product for use according to claim 6, wherein the patient is infected with SARS-COV-2.

8. The product for use according to any one of claims 1-5, wherein the patient is infected with influenza virus.

9. The product for use according to any one of the preceding claims, wherein the H1 receptor antagonist and the H2 receptor antagonist are administered together or simultaneously by injection, by nasogastric tube, orally, or mucosally.

10. The product for use according to any one of the preceding claims, wherein the H1 receptor antagonist and the H2 receptor antagonist are administered together or simultaneously by the patient PRO RE NATA (as needed), or sequentially at intervals ranging from 2 to 12 hours.

11. The product for use according to any one of the preceding claims, wherein the H1 receptor antagonist includes cetirizine and the H2 receptor antagonist includes famotidine.

12. The product for use according to any one of the preceding claims, wherein the H1 receptor antagonist is cetirizine at doses of 8.0-12 mg, and the H2 receptor antagonist is famotidine at doses of 15-24 mg.

13. The product for use according to any one of the preceding claims, wherein the patient is human.

14. The product for use according to any one of the preceding claims, wherein the treatment is carried out for from 3 to 30 days.

15. The product for use according to any one of the preceding claims, wherein the H1 receptor antagonist and the H2 receptor antagonist are administered as a unit dosage form containing both receptor antagonists.

## Patentansprüche

1. Präparat, umfassend einen H1-Rezeptorantagonisten und einen H2-Rezeptorantagonisten zur Verwendung in einem Verfahren zur: (a) Behandlung oder Verhinderung eines akuten Atemnotsyndroms oder von pulmonalen Beschwerden bei einem Patienten; oder (b) Verhinderung eines Krankenhausaufenthalts im Zusammenhang mit der Lunge, pulmonaler Intubation oder Lungenversagen bei einem Patienten; oder (c) Behandlung oder Verhinderung eines pulmonalen Zytokinsturms bei einem Patienten,
wobei das Präparat einem Patienten verabreicht wird, der mit einem Coronavirus oder einem Influenzavirus infiziert ist;
wobei der H1-Rezeptorantagonist Cetirizin, Levocetirizin, Diphenhydramin oder Gemische davon umfasst und wobei der H1-Rezeptorantagonist in einer Menge von 1,0 bis 14 mg pro Dosis, wie etwa einer Dosierung von 6,0 bis 14 mg, verabreicht wird; und
wobei der H2-Rezeptorantagonist Famotidin umfasst und wobei der H2-Rezeptorantagonist in einer Menge von 5,0 bis 28 mg pro Dosis, wie etwa einer Dosierung von 10 bis 28 mg, verabreicht wird.

2. Präparat, umfassend einen H1-Rezeptorantagonisten zur Verwendung in einem Verfahren zur: (a) Behandlung oder Verhinderung des akuten Atemnotsyndroms oder von Lungenbeschwerden bei einem Patienten; oder (b) Verhinderung eines Krankenhausaufenthalts im Zusammenhang mit der Lunge, pulmonaler Intubation oder Lungenversagen bei einem Patienten; oder (c) Behandlung oder Verhinderung eines pulmonalen Zytokinsturms bei einem Patienten,
wobei der H1-Rezeptorantagonist einem Patienten verabreicht wird, der mit einem Coronavirus oder einem Influenzavirus infiziert ist, und wobei dem Patienten ferner ein H2-Rezeptorantagonist verabreicht wird,
wobei der H1-Rezeptorantagonist Cetirizin, Levocetirizin, Diphenhydramin oder Gemische davon umfasst und wobei der H1-Rezeptorantagonist in einer Menge von 1,0 bis 14 mg pro Dosis, wie etwa einer Dosierung von 6,0 bis 14 mg, verabreicht wird; und
wobei der H2-Rezeptorantagonist Famotidin umfasst und wobei der H2-Rezeptorantagonist in einer Menge von 5,0 bis 28 mg pro Dosis, wie etwa einer Dosierung von 10 bis 28 mg, verabreicht wird.

3. Präparat, umfassend einen H2-Rezeptorantagonisten zur Verwendung in einem Verfahren zur: (a) Behandlung oder Verhinderung des akuten Atemnotsyndroms oder von Lungenbeschwerden bei einem Patienten; oder (b) Verhinderung eines Krankenhausaufenthalts im Zusammenhang mit der Lunge, pulmonaler Intubation oder Lungenversagen bei einem Patienten; oder (c) Behandlung oder Verhinderung eines pulmonalen Zytokinsturms bei einem Patienten,
wobei der H2-Rezeptorantagonist einem Patienten verabreicht wird, der mit einem Coronavirus oder einem Influenzavirus infiziert ist, und wobei dem Patienten ferner ein H1-Rezeptorantagonist verabreicht wird,
wobei der H1-Rezeptorantagonist Cetirizin, Levocetirizin, Diphenhydramin oder Gemische davon umfasst und wobei der H1-Rezeptorantagonist in einer Menge von 1,0 bis 14 mg pro Dosis, wie etwa einer Dosierung von 6,0 bis 14 mg, verabreicht wird; und
wobei der H2-Rezeptorantagonist Famotidin umfasst und wobei der H2-Rezeptorantagonist in einer Menge von 5,0 bis 28 mg pro Dosis, wie etwa einer Dosierung von 10 bis 28 mg, verabreicht wird.

4. Präparat zur Verwendung nach einem der vorhergehenden Ansprüche, wobei die Verwendung die Behandlung von akutem Atemnotsyndrom oder Lungenbeschwerden bei dem Patienten ist.

5. Präparat zur Verwendung nach einem der vorhergehenden Ansprüche, wobei die Verwendung die Behandlung eines pulmonalen Zytokinsturms bei dem Patienten ist.

6. Präparat zur Verwendung nach einem der vorhergehenden Ansprüche, wobei der Patient mit SARS-COV oder SARS-COV-2 infiziert ist.

7. Präparat zur Verwendung nach Anspruch 6, wobei der Patient mit SARS-COV-2 infiziert ist.

8. Präparat zur Verwendung nach einem der Ansprüche 1-5, wobei der Patient mit dem Influenzavirus infiziert ist.

9. Präparat zur Verwendung nach einem der vorhergehenden Ansprüche, wobei der H1-Rezeptorantagonist und der H2-Rezeptorantagonist gemeinsam oder gleichzeitig durch Injektion, nasogastrale Sonde, oral oder mukosal verabreicht werden.

10. Präparat zur Verwendung nach einem der vorhergehenden Ansprüche, wobei der H1-Rezeptorantagonist und der H2-Rezeptorantagonist gemeinsam oder gleichzeitig vom Patienten PRO RE NATA (bei Bedarf) oder sequentiell in Abständen im Bereich von 2 bis 12 Stunden verabreicht werden.

11. Präparat zur Verwendung nach einem der vorhergehenden Ansprüche, wobei der H1-Rezeptorantagonist Cetirizin umfasst und der H2-Rezeptorantagonist Famotidin umfasst.

12. Präparat zur Verwendung nach einem der vorhergehenden Ansprüche, wobei der H1-Rezeptorantagonist Cetirizin in Dosen von 8,0-12 mg ist und der H2-Rezeptorantagonist Famotidin in Dosen von 15-24 mg ist.

13. Präparat zur Verwendung nach einem der vorhergehenden Ansprüche, wobei der Patient ein Mensch ist.

14. Präparat zur Verwendung nach einem der vorhergehenden Ansprüche, wobei die Behandlung 3 bis 30 Tage durchgeführt wird.

15. Präparat zur Verwendung nach einem der vorhergehenden Ansprüche, wobei der H1-Rezeptorantagonist und der H2-Rezeptorantagonist als eine Einheitsdosisform, die beide Rezeptorantagonisten enthält, verabreicht werden.

## Revendications

1. Produit comprenant un antagoniste du récepteur H1 et un antagoniste du récepteur H2 pour une utilisation dans un procédé de : (a) traitement ou prévention d'un syndrome de détresse respiratoire aiguë ou d'une détresse pulmonaire chez un patient ; ou (b) prévention d'une hospitalisation liée à la fonction pulmonaire, d'une intubation pulmonaire ou d'une insuffisance pulmonaire chez un patient ; ou (c) traitement ou prévention d'une crise de cytokines pulmonaires chez un patient ;
dans lequel le produit est administré à un patient infecté par un virus corona ou un virus grippal ;
dans lequel l'antagoniste du récepteur H1 comprend la cétirizine, la lévocétirizine, la diphénhydramine, ou des mélanges de ceux-ci et dans lequel l'antagoniste du récepteur H1 est administré en une quantité de 1,0 à 14 mg par dose, telle qu'une dose de 6,0 à 14 mg ; et
dans lequel l'antagoniste du récepteur H2 comprend la famotidine et dans lequel l'antagoniste du récepteur H2 est administré en une quantité de 5,0 à 28 mg par dose, telle qu'une dose de 10 à 28 mg.

2. Produit comprenant un antagoniste du récepteur H1 pour une utilisation dans un procédé de : (a) traitement ou prévention d'un syndrome de détresse respiratoire aiguë ou d'une détresse pulmonaire chez un patient ; ou (b) prévention d'une hospitalisation liée à la fonction pulmonaire, d'une intubation pulmonaire ou d'une insuffisance pulmonaire chez un patient ; ou (c) traitement ou prévention d'une crise de cytokines pulmonaires chez un patient ;
dans lequel l'antagoniste du récepteur H1 est administré à un patient infecté par un virus corona ou un virus grippal, et dans lequel le patient reçoit en outre un antagoniste du récepteur H2,
dans lequel l'antagoniste du récepteur H1 comprend la cétirizine, la lévocétirizine, la diphénhydramine, ou des mélanges de ceux-ci et dans lequel l'antagoniste du récepteur H1 est administré en une quantité de 1,0 à 14 mg par dose, telle qu'une dose de 6,0 à 14 mg ; et
dans lequel l'antagoniste du récepteur H2 comprend la famotidine et dans lequel l'antagoniste du récepteur H2 est administré en une quantité de 5,0 à 28 mg par dose, telle qu'une dose de 10 à 28 mg.

3. Produit comprenant un antagoniste du récepteur H2 pour une utilisation dans un procédé de : (a) traitement ou prévention d'un syndrome de détresse respiratoire aiguë ou d'une détresse pulmonaire chez un patient ; ou (b) prévention d'une hospitalisation liée à la fonction pulmonaire, d'une intubation pulmonaire ou d'une insuffisance pulmonaire chez un patient ; ou (c) traitement ou prévention d'une crise de cytokines pulmonaires chez un patient ;
dans lequel l'antagoniste du récepteur H2 est administré à un patient infecté par un virus corona ou un virus grippal, et dans lequel le patient reçoit en outre un antagoniste du récepteur H1,
dans lequel l'antagoniste du récepteur H1 comprend la cétirizine, la lévocétirizine, la diphénhydramine, ou des mélanges de ceux-ci et dans lequel l'antagoniste du récepteur H1 est administré en une quantité de 1,0 à 14 mg par dose, telle qu'une dose de 6,0 à 14 mg ; et
dans lequel l'antagoniste du récepteur H2 comprend la famotidine et dans lequel l'antagoniste du récepteur H2 est administré en une quantité de 5,0 à 28 mg par dose, telle qu'une dose de 10 à 28 mg.

4. Produit pour une utilisation selon l'une quelconque des revendications précédentes, dans lequel l'utilisation traite le syndrome de détresse respiratoire aiguë ou la détresse pulmonaire chez le patient.

5. Produit pour une utilisation selon l'une quelconque des revendications précédentes, dans lequel l'utilisation traite une crise de cytokines pulmonaires chez le patient.

6. Produit pour une utilisation selon l'une quelconque des revendications précédentes, dans lequel le patient est infecté par SARS-COV ou SARS-COV-2.

7. Produit pour une utilisation selon la revendication 6, dans lequel le patient est infecté par SARS-COV-2.

8. Produit pour une utilisation selon l'une quelconque des revendications 1 à 5, dans lequel le patient est infecté par un virus grippal.

9. Produit pour une utilisation selon l'une quelconque des revendications précédentes, dans lequel l'antagoniste du récepteur H1et l'antagoniste du récepteur H2 sont administrés ensemble ou simultanément par injection, par sonde nasogastrique, par voie orale ou par voie mucosale.

10. Produit pour une utilisation selon l'une quelconque des revendications précédentes, dans lequel l'antagoniste du récepteur H1 et l'antagoniste du récepteur H2 sont administrés ensemble ou simultanément par le patient PRO RE NATA (selon les besoins), ou séquentiellement à des intervalles dans la plage de 2 à 12 heures.

11. Produit pour une utilisation selon l'une quelconque des revendications précédentes, dans lequel l'antagoniste du récepteur H1 comprend la cétirizine et l'antagoniste du récepteur H2 comprend la famotidine.

12. Produit pour une utilisation selon l'une quelconque des revendications précédentes, dans lequel l'antagoniste du récepteur H1 est la cétirizine à des doses de 8,0-12 mg, et l'antagoniste du récepteur H2 est la famotidine à des doses de 15-24 mg.

13. Produit pour une utilisation selon l'une quelconque des revendications précédentes, dans lequel le patient est humain.

14. Produit pour une utilisation selon l'une quelconque des revendications précédentes, dans lequel le traitement est effectué pendant 3 à 30 jours.

15. Produit pour une utilisation selon l'une quelconque des revendications précédentes, dans lequel l'antagoniste du récepteur H1 et l'antagoniste du récepteur H2 sont administrés sous forme d'unité de dosage contenant les deux antagonistes du récepteur.
